Europäisches Patentamt

European Patent Office

Office européen des brevets

(19)

(11) Publication number: 0 269 260
A2

# EUROPEAN PATENT APPLICATION

(12)

(21) Application number: 87309482.5

(22) Date of filing: 27.10.87

(51) Int. Cl.⁴: **C12Q 1/08** , C12N 15/00 , C12P 19/34

(30) Priority: 29.10.86 US 924911
30.04.87 US 44449
04.09.87 US 93453

(43) Date of publication of application:
01.06.88 Bulletin 88/22

(84) Designated Contracting States:
ES GR

(71) Applicant: **BIOTECHNOLOGY RESEARCH PARTNERS LIMITED**
**2450 Bayshore Parkway**
**Mountain View, California 94043(US)**

(72) Inventor: **Frossard, Philippe M.**
**580 Arastradero Road, No. 304**
**Palo Alto California 94306(US)**

(74) Representative: **Goldin, Douglas Michael et al**
**J.A. KEMP & CO. 14, South Square Gray's Inn**
**London WC1R 5EU(GB)**

(54) apoAI-CIII-AIV, apoAII apoB, apoCI, and LDL receptor polymorphisms for genetic fingerprinting and predictive of atherosclerosis.

(57) The invention offers a method of genetic analysis which is potentially an early detection method for atherosclerosis. The method comprises detecting the presence or absence of polymorphic states correlated with this disease which are proximal to the apolipoprotein AI-CIII-AIV gene complex, or to the apoAII, apoB, apoCI or, LDL-R genes. Individuals with particular polymorphic sites are likely to experience severe atherosclerotic symptomologies.

EP 0 269 260 A2

## apoAI-CIII-AIV, apoAII apoB, apoCI, AND LDL RECEPTOR POLYMORPHISMS FOR GENETIC FINGERPRINTING AND PREDICTIVE OF ATHEROSCLEROSIS

Technical Field

The invention relates to the use of genetic polymorphisms to determine disease states. More particularly, the invention concerns the use of polymorphisms of the apolipoprotein AI-CIII-AIV gene region, and of the apoB, apoCI, apoAII and LDL receptor genes, to diagnose susceptibilities to atherosclerosis.

Background Art

The degree of morbidity and mortality associated with atherosclerosis in developed countries is higher than that associated with any other particular disorder, even cancer. The disorder manifests itself in the form of cholesterol deposition in arterial cell walls. The deposition is slow and irreversible and starts at an early age. Clinical symptoms may take years to manifest themselves and are extremely serious; they include coronary heart disease and stroke. Generally, the disease process will have begun long before these clinical manifestations appear.

Because environmental as well as hereditary factors influence the course of the cholesterol deposition and offer means for at least a mitigation of the process, it is desirable to have available à diagnostic technique which provides an early warning of the onset of the deposition. The present technique depends on measuring cholesterol or triglyceride levels in serum, and while these levels are, indeed, capable of accurate measurement, they do not offer the desirable high correlation to true susceptibility. More reliable predictive methods, which rely on detection of atheromatous lesions, use highly invasive procedures, which are sufficiently painful and expensive that they cannot be employed on a screening basis, or even applied to specific groups selected on the basis of family histories. These techniques also offer too little, too late; by the time the atheromatous lesions have appeared, the most effective time for treatment has been passed.

The importance of early detection is made more poignantly evident by the fact that an effective, but · inconvenient and unattractive long term treatment is available--i.e., lowering serum cholesterol through consistently controlled diet or use of cholesterol-lowering drugs. Resistance to this approach will be encountered unless it is clear that the "deprivation" is warranted. The problem is not what the treatment should be, but to whom the treatment should be applied.

A technique that inherently offers the advantages of early detection, if its results can be effectively correlated with the disorder to be assessed, is genetic analysis. Since the genomic characteristics of an individual are basically determined, it is assumed, at conception, genetic aberrations which are indicia of later metabolic disorders are an ideal early diagnosis tool. Genetic testing can be routinely conducted using present methodology, as early as the seventh week of fetal life. Over the last ten years, the availability of restriction enzymes and DNA probing techniques has made possible the use of "restriction fragment length polymorphisms" (RFLPs) in such diagnosis. Using the by now well established Southern blot hybridization technique (Southern, E., J Mol Biol (1975) 98:503-517), it has been possible successfully to diagnose sickle cell anemia (Kan, Y.W., et al, Proc Natl Acad Sci (USA) (1978) 75:5631); β-thalassemia (Antonarakis, S.E., et al, Proc Natl Acad Sci (USA) (1983) 79:137); type II diabetes (Rotwein, P., et al, Science (1981) 213:1117); familial growth hormone deficiency (Phillips, J.A., III Banbury Report 14, Cold Spring Harbor Laboratory (1983) pp 305-315); phenylketonuria (Woo, S.L.C., et al, Nature (1983) 306:151); Huntington's disease (Gusella, J.F., et al, Nature (1983) 306:234); and hemophilia B (Gianelli, et al, Lancet (1984) i:239, Grunenbaum, et al, J Clin Invest (1984) 73:1491).

All the foregoing successes are grounded in the identification of a particular polymorphism or polymorphisms which correlates with the disease or disorder in question. It should be noted, however, that the technique is also useful from a more general standpoint in genetically fingerprinting individuals, e.g. in paternity testing or the like.

It has been calculated that the number of polymorphisms expected in the human genome should be of the order of $10^7$, based on an assumed probability of 0.05 for a given nucleotide to be polymorphic; this number being inferred from studies of the human growth hormone, αl-antitrypsin and β-like globin gene cluster loci (Jeffreys. A. J., Cell (1979) 18:1-10; Oster, H., et al, Am J Hum Gen (1984) 36(suppl) 150S). In detecting or predicting disease, the challenge is to determine which of these over ten million polymor-

0 269 260

phisms is associated with a particular disorder, and to devise a procedure to detect it. The present invention has met this challenge with regard to atherosclerosis, and has also identified a pattern of polymorphisms associated with the same locations in the genome useful in characterizing particular individuals and patterns of inheritance.

Disclosure of the Invention

The invention provides identification of polymorphisms which are predictive of the subsequent development of atherosclerosis or which are indicative of protection against development of this disease, and include polymorphisms in the regions of the genome which encode apolipoproteins AI, CIII and AIV (the apoAI-CIII-AIV gene complex), (and which encode apolipoproteins apoAII, B, and CI (the apoAII, apoB and apoCI genes). The invention also includes identification of polymorphisms in the region of the genome encoding the receptor for low density lipoprotein (LDL-R gene). The apoAI-CIII gene region of the apoAI-CII-AIV complex is described in applicant's copending applications U.S. Serial Nos. 782,666, filed 30 September 1985, 900,593, filed 26 August 1986, 924,911, filed 29 October 1986, and 044,449, filed 30 April 1987. The disclosure of each of these co-pending applications is hereby incorporated by reference.

The polymorphisms of the present invention, taken together or in subsets, can provide an identity profile of a particular individual, thus providing a means to follow family inheritance patterns and to assess relationships between individuals. In addition, since these polymorphisms are located in the genomic sequences which regulate lipid metabolism, is is probable that their pattern of presence or absence will show some additional specific, as yet unknown, correlation to symptomology.

In one aspect, then, the invention is directed to a method of genetically identifying an individual, in particular with respect to predicting the likelihood of development of atherosclerosis in said individual, which method comprises:

detecting the presence or absence of a polymorphic site in the third intron of the apolipoprotein AI (apoAI) gene detectable in a BanI digest (the "BanI-apoAI" polymorphism); and/or

detecting the presence or absence of a polymorphic site in the first intron of the apoAI gene detectable in an MspI digest (the "MspI/0.73-apoAI" polymorphism); and/or

detecting the presence or absence of a polymorphic site in the third intron of the apoAI gene detectable in a BanII digest (the "BanII-apoAI" polymorphism); and/or

detecting the presence or absence of a polymorphic site in the third intron of the apoAI gene detectable in a BsmI digest (the "BsmI-apoAI" polymorphism); and/or

detecting the presence or absence of a polymorphic site 1.0 kb 3′ of the apoAI gene detectable in a BstXI digest (the "BstXI-apoAI" polymorphism), and/or

detecting the presence or absence of a polymorphic site at the apoAII gene locus detectable in a BstEII digest ("the BstEII-apoAII" polymorphism), and/or

detecting the presence or absence of a polymorphic site at the apoB gene locus detectable in an HaeIII digest (the HaeIII-ApoB polymorphism), and/or

detecting the presence or absence of a polymorphic site at the apoB gene locus detectable in an MvaI digest ("the MvaI-ApoB" polymorphism), and/or

detecting the presence or absence of a polymorphic site at the apoB gene locus detectable in an NsiI digest ("the NsiI-ApoB" polymorphism), and/or

detecting the presence or absence of a polymorphic site at the apoB gene locus detectable in an HpeI digest (the "HpaI-ApoB" polymorphism), and/or

detecting the presence or absence of a polymorphic site at the apoCI gene locus detectable in a BglI digest (the "BglI-apoCI" polymorphism), and/or

detecting the presence or absence of a polymorphic site at the apoCI gene locus detectable in a DraI digest (the "DraI-apoCI") polymorphism), and/or

detecting the presence or absence of a polymorphic site at the apoCI gene locus detectable in a TaqI digest (the "TaqI-apoCI" polymorphism), and/or

detecting the presence or absence of a polymorphic site at the LDL-R gene locus detectable in a Cfr13I digest (the "Cfr13I-LDL" polymorphism), and/or

detecting the presence or absence of a polymorphic site at the LDL-R gene locus detectable in a BstEII digest (the "BstEII-LDL" polymorphism).

Stated alternatively, the invention is directed to a method for genetic fingerprinting, in particular, detecting susceptibility to atherosclerosis which comprises:

detecting a 1.46 kb vs. both a 0.82 kb and a 0.64 kb BanI digestion fragment hybridizing to the apoAI[1]

3

probe, or its substantial equivalent; and/or

detecting a 0.73 kb vs . 0.65 kb MspI digestion fragment hybridizing to the apoAI[1] probe, or its substantial equivalent; and/or

detecting a 0.452 kb vs . 0.274 kb BanII digestion fragment hybridizing to the apoAI[2] probe, or its substantial equivalent; and/or

detecting a 6.9 kb vs . 5.6 kb BsmI digestion fragment hybridizing to the apoAI[1] probe, or its substantial equivalent, or the presence or absence of a 9.3 kb fragment hybridizing to apoCIII probe or its substantial equivalent; and/or

detecting a 4.5 kb vs. 1.8 kb BstXI digestion fragment hybridizing to the apoCIII probe, or its substantial equivalent, or a 4.5 kb vs. 2.7 kb BstXI digestion fragment hybridizing to an apoAI[1] probe or its substantial equivalent; and/or

detecting a 4.8 kb vs. 6.5 kb BstEII digestion fragment hybridizing to the apoAII probe, or its substantial equivalent; and/or

detecting an HaeIII digestion fragment of variable size between 0.9 kb and 1.1 kb hybridizing to the 3'-apoB probe or its substantial equivalent; and/or

detecting a 0.96 kb vs. 0.76 kb MvaI digestion fragment hybridizing to the 3'-apoB probe or its substantial equivalent; and/or

detecting a 3.8 kb vs. 6.2 kb NsiI digestion fragment hybridizing to the 5'-apoB probe, or its substantial equivalent; and/or

detecting the presence or absence of a 15.1 kb HpaI digestion fragment hybridizing to the 5' apoB probe or its substantial equivalent; and/or

detecting an 8.7 kb vs. 6.2 kb BglII digestion fragment hybridizing to the apoCI probe, or its substantial equivalent; and/or

detecting a 10.2 kb vs. both a 7.6 kb and a 2.6 kb DraI digestion fragment hybridizing to the apoCI probe, or its substantial equivalent; and/or

detecting the presence or absence of a 5.9 kb TaqI digestion fragment hybridizing to an apoCI probe or its substantial equivalent; and/or

detecting a 3.0 kb vs. 4.0 kb Cfr13I digestion fragment hybridizing to the LDL-R probe or its substantial equivalent; and/or

detecting both a 22 and 11 kb vs. a 33 kb BstEII digestion fragment hybridizing to the LDL-R probe, or its substantial equivalent.

The invention is also directed to kits suitable for performing the methods of the invention.

## Brief Description of the Drawings

Figure 1 shows a map of twelve polymorphisms found in the apoAI-CIII-AIV gene complex.

Figure 2 gives the DNA sequence for the apoB probe used in detecting some of the polymorphisms disclosed herein.

## Modes of Carrying Out the Invention

In the description below, distances of polymorphisms from reference points and lengths of deletions are often given in bp or kb. Where the sequence is known, such measures can be quite precise, but when assessed by measuring fragment sizes on gels or by other experimental means, these measures contain a margin of uncertainty, as is well understood in the art. In general, for measures of >4 kb, the margin of uncertainty is ± ~ 0.3 kb; for smaller lengths, the error is ± ~ 10%. Thus, the "300 bp" deletion may be slightly larger or smaller, and the 4 kb spacing from the apoAI gene is only approximate.

The nomenclature used to identify the polymorphisms disclosed herein is as follows. Generally, each polymorphism is identified with the restriction enzyme that cleaves a particular segment of the genome to give either a "normal" (more common allele) or a "polymorphic" (less common allele) fragment.

Where digestion with a given restriction enzyme yields more than one polymorphism, each polymorphism is then also identified by the length of the polymorphic fragment--e.g., "MspI/0.73 apoAI".

Related polymorphisms not discussed at length herein are disclosed in applicant's copending applications previously incorporated by reference.

A. Techniques for Detection of Polymorphisms

Application of the method of the invention to predict potential atherosclerotic individuals or to obtain a genetic "fingerprint" based on some or all of the polymorphisms associated with the general genomic apoAI-CIII-AIV complex and apoB and CI genes, employs standard techniques of DNA extraction, purification, restriction enzyme digestion, and size separation. Techniques for hybridization using probes and successful detection of hybridized substrate arranged according to molecular weight are also well known to those in the art. The general approach to finding and detecting the significant polymorphisms is the following:

DNA is extracted from the somatic cells of the individual to be tested, for example from leukocytes, placental cells, cultured fibroblasts, or, in the case of fetal individuals, from cells of the amniotic fluid. The high molecular weight DNA fraction is separated, and subjected to treatment with a particular, selected restriction enzyme, such as, for example, EcoRI, BamHI, MstI, XmnI, and the like. After digestion of the high molecular weight DNA, the digest is applied to a polyacrylamide or agarose gel and subjected to electrophoresis to obtain separation of the DNA fragments resulting from restriction enzyme digestion into positions on the gel determined by the size (length) of the fragment. The contents of the gel are then replicated by transferring to a nitrocellulose filter or other suitable matrix for use as a probe hybridization support. The DNA fragments, either before or after transfer to the nitrocellulose filter replica, are treated with a denaturant such as sodium hydroxide/salt. The denatured, single-stranded DNA, replicated electrophoresis patterns are probed with labeled (usually by $^{32}$P) single-stranded DNA fragments. Other labels besides radioactivity, such as fluorescent molecules may also be used. The complex formed with probe may also be detected by methods designed to detect the complex per se, rather than to label either component, such as, for example, binding to labeled antibodies specific for double-stranded nucleic acids.

When probes are labeled by nick translation using, for example, $\alpha[^{32}P]$ dCTP and $\alpha[^{32}P]$ dGTP, which results in fragmentation of the probe, cDNA probes which are complementary only to the exon regions of the gene and which span over intron regions are readily workable in the method of the invention.

Depending on the probe selected, fragments will be detected which derive from a particular region on the genome. For example, in the method of the invention, a cDNA sequence from the apolipoprotein AI (apoAI), apolipoprotein AII (apoAII), apolipoprotein B (apoB), apolipoprotein CI (apoCI), or LDL receptor (LDL-R) gene sequences is used as a probe. Therefore, the only fragments which will appear on the hybridized filters are those which contain sequences complementary to this probe--i.e., only those which have not been severed either in the genome itself or by the restriction enzyme cleavage from the complementary apoAI, apoAII, apoB, apoCI or LDL receptor fragment. Stated in another way, by using a particular probe, alterations in the genome which are proximal to sequences corresponding to that probe are detected.

The specific procedures used in the general process described in the preceding paragraphs are understood in the art. Procedures for DNA extraction from somatic cells may, for example, be found in Kan, Y.W., et al, Proc Natl Acad Sci (USA) (1978) 75:5631-5635; Taylor, J.M., et al, Nature (1984) 251:392-393, and Kan, Y.W., et al, N Eng J Med (1977) 297:1080-1084. Further improvements which permit rapid extraction of the DNA are also disclosed by Law, D. G., et al, Gene (1984) 28:153-158. Techniques for size separation of the restriction enzyme treated DNA fragments are also described in the foregoing references. Restriction enzyme digestions are generally standard in the art and are carried out under buffer, ionic strength, and temperature conditions which are specified by the manufacturer of the particular restriction enzyme.

Transfer to nitrocellulose or other support and probing by prehybridization with nonspecific DNA followed by hybridization with labeled probe are also standard procedures disclosed, for example, in the foregoing references and by Southern, E., (supra). The section of the genome which is fingerprinted or otherwise subject to study using the results is, of course, dependent on the nature of the probe. The probes useful in the present invention are selected from the apoAI-CIII-AIV gene complex, and from the apoAII, apoB, apoCI, and LDL receptor genes.


B. Nature of the Probes Useful in the Invention

The fragment pattern obtained is diagnostic for a particular polymorphism if the probe selected is complementary to a DNA sequence sufficiently proximal to the polymorphism on the genome that it is not severed from the polymorphism by the restriction cleavage, and has a low probability of being segregated from the polymorphism by crossing over. Acceptable distance limits between the region of probe complementarity and the polymorphism are therefore arbitrary. Generally, probes which hybridize to DNA sequences within 10 kb upstream or downstream of the polymorphism give acceptable results. Occasionally, the pattern of restriction enzyme cleavage may place a distal probe hybridization site on a fragment irrelevant to the polymorphism. The closer the probe to the polymorphism, the greater the range of usable restriction enzymes. Accordingly, as used herein, a probe which is a "substantial equivalent" to a specified probe is one which hybridizes to the same fragment length in digests of DNA from individuals with a particular polymorphism when the same restriction enzyme is used. For example, in the detection of the XmnI/8.2 kb polymorphism of the apoAI gene, used as a model for atherosclerosis-related polymorphisms, two probes described below, an apoAI probe and a p5'AI probe, are equivalent when XmnI is used as a restriction enzyme. For detection of this polymorphism using RsaI digestion, they are not. (Of course, the designated probe can be modified in a trivial manner by being made longer or shorter or by selecting a slightly displaced sequence.)

Since atherosclerosis is associated with a defect in cholesterol metabolism, the apoAI-CIII-AIV gene complex which is associated with regulation of blood plasma cholesterol is a region of appropriate probe complementarity. Regulation of blood plasma cholesterol is related to high density lipoprotein (HDL), which is known to be a carrier of cholesterol in the bloodstream, and thus believed to play a significant role in regulating its level—apoAI is the major structural protein of HDL. It is a 243 amino acid polypeptide having a molecular weight of 28,000. In addition to being a structural member of the HDL complex, apoAI is also an activator of lecithin-cholesterol acyltransferase, which enzyme is responsible for transforming the lamellar surface structures of chylomicrons into pseudomicellar spherical HDL by de novo synthesis of cholesterol esters and lysolecithin. The apoAI protein may also be the HDL component recognized by the HDL receptors.

Accordingly, the apoAI gene region is a portion of the genome where modification might occur in individuals with a prognosis for atherosclerosis or related disorders. This gene lies close to a related gene encoding apolipoprotein CIII (apoCIII) which is 2.5 kb from the apoAI gene on the long arm of chromosome 11 (Cheung. p., et al, Proc Natl Acad Sci (USA) (1984) 81 :508-511; Law, S. W., et al, Biochem Biophys Res Comm (1984) 118:934-942). The apoAIV gene is located 7.8 kb 3' to the apoCIII gene and 12.5 kb 3' to the apoAI gene (Karathanasis, S.K., Proc. Natl. Acad. Sci. USA (1985) 82:6374-6378). This region of the genome is herein designated the "apoAI-CIII-AIV complex". Genomic and cDNA clones for apoAI, apoCIII and apoAIV have been isolated by, e.g., Karathanasis, S. K., et al, Proc Natl Acad Sci (USA) (1983) 80:6147-6151; Shoulders, S. C., et al, Nucleic Acids Res (1983) 11:2827-2837 and by others. Various apoAI and apoCIII probes have been described (see, e.g., Karathanasis, S.K., et al (supra); Shoulders, S.C., et al (supra)) and have been used previously as hybridization probes to detect polymorphisms.

Karathanasis, S. K., et al, (Nature (1983) 301:718-720; ibid. (1983) 305:823-825) showed a 6.5 kb DNA insertion in the coding region of apoAI in one family suffering from HDL deficiency. Rees, A., et al, Lancet - (1983) i:444-446 showed an SstI polymorphism in the 3' flanking region of the apoCIII gene which was present in some hypertriglyceridemic patients. Seilhamer, J.J., et al, DNA (1984) 3:309-317 have disclosed an MspI polymorphis, in the third intron of the apoAI gene.

Apolipoprotein AII is a 77 amino acid polypeptide and is a component of HDL. Although the functional role of apoAII remains unclear, it might regulate the activities of lecithin-cholesterol acyltransferase (Scanu, A.M., et al, Ann NY Acad Sci (198_) 348:160-173) and hepatic lipase (Shinomiya, M., et al, Biochim Biophys Acta (198_) 713:292-299; Scott, Y., et al, Lancet (1985) 1:770-773); have described the presence of an MspI polymorphic site 3' to the apoAII gene, which correlates with increased serum apoAII levels.

Apolipoprotein B is the major protein component of very low density lipoproteins (VLDL) and of chylomicrons. It is the sole protein in low density lipoproteins (LDL), and is essential for the assembly and secretion of chylomicrons and VLDL. It also functions as the ligand for removal of LDL from circulation by receptor-mediated uptake into a variety of cells. (Lusis, A.J., et al, Proc Natl Acad Sci (USA) (1985) 82: 4597-4601.) Four major plasma species of apoB have been described (Kane, J.p., et al, Proc Natl Acad Sci (USA) (1980) 77: 2465-2469). However, two of these appear to arise from one of the others by virtue of the protease activities found in plasma. (Cardin, A.D., et al, J Biol Chem (1984) 259: 8522-8528; Yamamoto, M., et al, J Biol Chem (1985) 260: 8509-8513). One of the primary forms, apoB-48, is synthesized by the intestine and is a component of chylomicrons; the other primary form, which is apparently attacked by the plasma protease, apoB-100, is the protein ligand on LDL that binds to the LDL receptor and results in uptake and catabolism of LDL by the liver (Deeb. S.S., et al, Proc Natl Acad Sci (USA) (1985) 82: 4983-4986). In any event, the apolipoproteins encoded by the apoB gene are integral to cholesterol and fat metabolism. Indeed, it has been shown by others that elevated plasma levels of apoB-100 have been found in individuals with premature coronary artery disease (Brunzel. J.D., et al, Atherosclerosis (1984) 4: 79-83). and individuals with familial hyperlipidemia and hypercholesterolemia also seem to have elevated levels of this protein (Brunzel, J.D., et al, ibid.; Brunzel, J.D., et al, J Lipid Res (1983) 24: 147-155). At least partly because of this interest, cDNA clones for apoB or portions thereof have been prepared (Deeb, S.S., et al, and Lusis, A.J., et al, both supra; Protter, A.A., et al, Proc Natl Acad Sci, in press). Thus, a portion of the apoB gene is useful as a probe to detect polymorphisms in this area, as will be described.

The apoCI gene encodes the major protein component of human very low density lipoprotein, and a minor component of high density lipoprotein. These factors are also intimately involved in lipid metabolism. In addition, apoCI binds to phospholipid and can activate lecithin cholesterol acyltransferase (L-CAT) in vitro in a manner similar to that of apoAI. Thus, apoCI, like the apoAI-CIII-AIV complex, is intimately involved in cholesterol metabolism (Davison P.J., et al, Biochem Biophys Res Comm (1986) 136: 876-884). In addition, it is shown in the Davison paper that there appear to be two apoCI gene loci which are located on the same chromosome. These loci apparently have sufficient homology that a standard apoCI probe would bind to either.

Thus, also useful as a probe is the apoCI region, which encodes apolipoprotein CI. A suitable probe for detection of polymorphisms in this region is the "apoCI" probe, a 300 bp apoCI cDNA isolated from a fetal liver library, and having a sequence exactly identical to that contained in the sequence published by Knott, T.J., et al, Nucleic Acids Res (1984) 12:3909-3915, shown in Figure 3 thereof.

As discussed above, LDL receptor mediates uptake of LDL from the circulation. Elucidation of LDL receptor function may facilitate understanding the mechanism of diseases associated with defective receptor function, such as atherosclerosis. Human low density lipoprotein receptor (LDL-R) DNA has been isolated (Yamamoto et al, Cell (1984) 39:27-38), and a probe may be obtained as described in detail below.

The apoAI[1] ,apoAI[2] , 5'AI, apoCIII, apoAII, 5'-apoB, 3'-apoB, apoCI, and LDL-R probes which illustrate those useful in the invention are described in detail below. Any probes substantially equivalent to these for a specific restriction enzyme, as defined above, may be used.

The relationship of the probe/enzyme combination to results can be illustrated using a known atherosclerosis predictive polymorphism, which consists of an approximately 300 bp deletion about 4 kb 5' of the apoAI gene. The deletion is detectable, for example, by digesting the extracted cellular DNA with XmnI, subjecting the fragments to electrophoresis gel separation, and probing with either apoAI or p5'AI probes. All persons showing an 8.2 kb fragment are at high (perhaps 100%, risk of developing atherosclerosis, if, indeed, the symptoms have not already appeared. Because of the pattern of XmnI restriction sites in this region, the apoAI and p5'AI probes are substantial equivalents in this test. Based on this pattern, one would expect that substantially equivalent probes would be found within 10 kb of the polymorphism. i.e., within about 14 kb upstream or 4 kb downstream of the apoAI gene with respect to digestion with XmnI. These two probes are, however, not equivalent with respect to a series of additional restriction enzymes which are capable of detecting this polymorphism but which cut either closer to the polymorphism or on the side of it opposite the apoAI gene. For such restriction enzymes, probes equivalent to p5'AI must be located between the restriction site and the polymorphism.

## C. Atherosclerosis-Related Polymorphisms

### The XmnI/8.2 kb Polymorphism

One abnormal allele associated with atherosclerosis, i.e., that containing the abovementioned 300 bp deletion, results in an 8.2 kb XmnI digest fragment, detectable with the first apoAI probe or with the p5'AI probe, rather than the 8.5 kb fragment which is obtained "normally". The definition of "normal" in the foregoing sense has to do with the nature of the disease being diagnosed and describes a genomic makeup which does not dispose the subject to the disorder. The majority of the total population will be "normal".

To correlate the genetic test results with atherosclerosis, 125 individuals were tested using XmnI and apoAI or p5'AI probe. DNA from ten of these individuals contained the 300 bp deletion, i.e., yielded the 8.2 kb fragment. All of these ten had previously suffered a myocardial infarction; furthermore the presence of the deletion correlated with low levels of HDL cholesterol, the major feature of familial combined hyperlipidemia, a disease associated with early development of severe atherosclerosis and its subsequent clinical symptoms. Twenty-five of the individuals tested were normal control healthy individuals; the DNA from none of these persons contained the deletion. The remaining ninety tested individuals (also not showing the 8.2 kb fragment) were known to have atheromatous plaques as detected by angiography, but not all had suffered heart attacks. Thus, in this sample the deletion is present only in individuals at serious risk from atherosclerosis. It is not present in all such individuals. Therefore, a positive test using this polymorphism is diagnostic and predictive and preventive methods of therapy would be clearly appropriate. (Of course, a negative test may not mean that the individual is at no risk; it may merely indicate that other genetic defects or other factors predispose that individual to the atherosclerosis problem.)

D. Additional Polymorphisms

The polymorphisms of the invention associated with the apoAl-CIII-AIV complex or with the apoAll, apoB, apoCl or LDL-R gene loci may also be analyzed using a group of individuals similar to that discussed in ¶C above. This group is divided into atherosclerosis "patients" --i.e., those persons shown to have atheromatous plaques as detected by angiography, whether or not they had heart attacks; and "controls", which consist of those with negative results in this test. Use of this division permits statistical analysis of data to assign increased or decreased risk of atherosclerosis, depending on the presence or absence of particular polymorphisms.

The polymorphisms of the invention are set forth along with the XmnI/8.2 kb polymorphism in Table 2 of the examples below. Some of these polymorphisms have been characterized in terms of their location; however, all can be defined operationally as detectable by digestion by a particular restriction enzyme and probing with a specific probe or its substantial equivalent. The pattern of the presence or absence of these polymorphisms is capable of fingerprinting an individual genetically so that hereditary patterns between parents and progeny can be traced. Also, because these polymorphisms are associated with regions of the genome important in lipid metabolism and transport, the presence of one or, more probably, several of these polymorphisms is likely to be associated with a propensity to disorders of lipid metabolism and related symptomologies. At the present time, limited precise correlations have been made between the presence of particular polymorphisms in this group and particular symptomologies. However, ascertaining the presence or absence of these genetic modifications is useful for the general purpose of identifying individuals with presumptively high risk of lipid disorder symptomologies and for providing a genetic characterization of individuals and family patterns.

E. Kits

The reagents suitable for applying the method of the invention to detect the appropriate polymorphisms may be packaged into convenient kits providing the necessary materials, packaged into suitable containers, and, optionally, suitable containers or supports useful in performing the assay. The essential components of the assay include the restriction enzyme associated with the polymorphism, and a suitable probe. Additionally, packages containing concentrated-forms of reagents used for hybridization, prehybridization, DNA extraction, etc. may be included if desired. In particular, however, labeled probe, or reagents suitable to form conveniently labeled probe, are useful in facilitating the conduct of the method of the invention.

F. Examples

The following examples are specific with respect to the probes exemplified and with respect to the precise conditions of DNA extraction, probe hybridization, etc. It is understood that these factors are illustrative but not limiting. The essential features of the invention as it relates to detection of a particular polymorphism are selection of enzyme and probe. For example, in detecting the standard XmnI/8.2 deletion, one may use XmnI digestion of the genomic DNA and probe with a sequence complementary to the genomic sequence (in the nonrepeating regions) proximal (i.e., within ~ in this case < 8.2 kb) to the site of the polymorphism. Alternatively, other restriction enzymes may be used in conjunction with a probe which hybridizes in particularly close proximity to the polymorphism.

The various polymorphisms of the invention may be detected using other specific restriction enzymes than those illustrated below. A variety of substantially equivalent probes could be designed with respect to these regions, and the particular restriction enzyme and cDNA probe combination chosen below are arbitrary. However, it should be noted, as is understood in the art, that the efficacy of the probe is enhanced as it moves closer to the site of the polymorphism. Otherwise, additional, cleavage points may be encountered between the polymorphism and the probe, and also the probing site may be separated from the site of the polymorphism during replication by crossing-over events.

8

## F.1. Procedures for Analysis

Leukocytes were obtained from freshly drawn blood collected from each of the human subjects, and high molecular weight genomic DNA was isolated by the procedure of Law, D. J., et al, Gene (1984) 28:153-158.

High molecular weight DNA was divided into portions and each was digested to completion with one of the various restriction enzymes under conditions recommended by the suppliers (New England Biolabs and Bethesda Research Laboratories). The digests were electrophoresed in horizontal agarose gels in 30 mM $NaH_2PO_4$, 36 mM Tris, 1 mM EDTA, pH 7.7. After electrophoresis, DNA fragments were denatured in situ in 0.5 M NaOH/1.5 M NaCl for 2 × 10 min, neutralized in 1 M ammonium acetate pH 7.2 for 2 × 10 min, and transferred overnight onto nitrocellulose paper (Schleicher and Schuell). The filters were rinsed in 2 × SSC (1× SSC is 0.15 M NaCl, 0.015 M sodium citrate pH, 7.4) and baked for 2 hr at 80°C in vacuo and then were prehybridized for 5 hr in plastic bags using 0.3 ml/cm² of a solution containing 5× SSPE (1× SSPE is 10 mM Na phosphate pH 7.4, 0.18 M NaCl and 1 mM EDTA) containing 5× Denhardt's solution (1 × Denhardt's contains 0.2 mg/ml each of Ficoll, polyvinylpyrrolidone and bovine serum albumin), 40% (vol/vol) formamide, and 250 μg/ml sheared and denatured salmon sperm DNA, and hybridized overnight in the same bag in 0.1 ml/cm² of 5× SSPE, 1× Denhardt's solution, 40% (vol/vol) formamide, 10% dextran sulfate, and 100 μg/ml sheared and denatured salmon sperm DNA, mixed with 100 ng per bag (containing 1 or 2 filters) of the appropriate 32P-labeled probe, as discussed below. Prehybridization and hybridization were performed at 42°C.

Filters were then washed twice at room temperature in 2× SSC and twice at 65°C in 2× SSC. 1× Denhardt's solution. DNA sequences hybridized to the 32P-labeled probes were visualized by autoradiography using XAR-5 films (Kodak) and Cronex intensifying screens (Dupont) at -70°C for 18 hr to 2 days.

Various specific probes are relevant to the illustrations below: a "first" apoAI probe designated apoAI[1], a "second" apoAI probe designated apoAI[2], p5'AI probe, and apoCIII probe relevant to the apoAI/CIII/AIV complex; apoAII probe; 5'-apoB probe and 3'-apoB probe, relevant to apoB; apoCI; and pLDLR-3 probe.

The apoAI[1] probe is a 600 bp cDNA fragment which had been cleaved with AvaII to remove terminal A/T tails, as described by Seilhamer, J.J., et al, DNA (1984) 3:309-317 (supra) incorporated herein by reference. The probe was labeled to a specific activity of 2.0-5.0 × 10⁸ cpm/μg by nick-translation, using the BRL nick-translation kit (Bethesda Research Laboratories) under recommended conditions with α [32p] dGTP and α [32P] dCTP (800 Cl/mmol; Amersham Corporation) in the presence of unlabeled dATP and dTTp. The probe was denatured just before the hybridization step by incubation for 5 min in a boiling water bath, followed by rapid cooling in ice water.

The apoAI[2] probe (also sometimes referred to herein as "HSAI probe") is a 1.0 kb HindIII/SstI cDNA subfragment prepared from a DNA fragment isolated from a human genomic library. The isolated fragment contained, in order, 6 kb of sequence 5' to the apoAI gene, the entire apoAI gene, 2.6 kb of intergenic region between the apoAI and apoCIII gene, and 1.6 kb of apoCIII gene. This fragment was digested with HindIII and SstI and smaller fragments isolated. The 1.0 kb HindIII/SstI fragment designated apoAI[2] resulting from the digestion process corresponds to the 5' half of the apoAI gene.

The p5'AI probe was prepared as follows. A 12.5 kb EcoRI/EcoRI fragment was isolated from a human genomic library as described above. This fragment was digested with HindIII and SstI as above. A 1.4 kb SstI/HindIII fragment resulting from this digestion contained a unique sequence appropriate as a hybridization probe and was designated p5'AI. It was labeled as described above. The p5'AI probe hybridizes close to the atherosclerosis deletion polymorphism described below, which is 4 kb 5' of the apoAI gene. Restriction endonucleases other than XmnI could thus be used to detect this deletion, some giving fragments of smaller length which are more easily separated on agarose gel and easier to visualize.

The apoCIII probe is a 480 bp SphI/pvuII partial cDNA segment described by Levy-Wilson, B., et al, DNA (1984) 3:359-364.

The apoAII probe is a cDNA of 281 bp having the sequence shown as nucleotide #12 to nucleotide #290 in the sequence disclosed by Moore, et al, Biochem Biophys Res Comm (1984) 123:1-7, (supra), incorporated herein by reference.

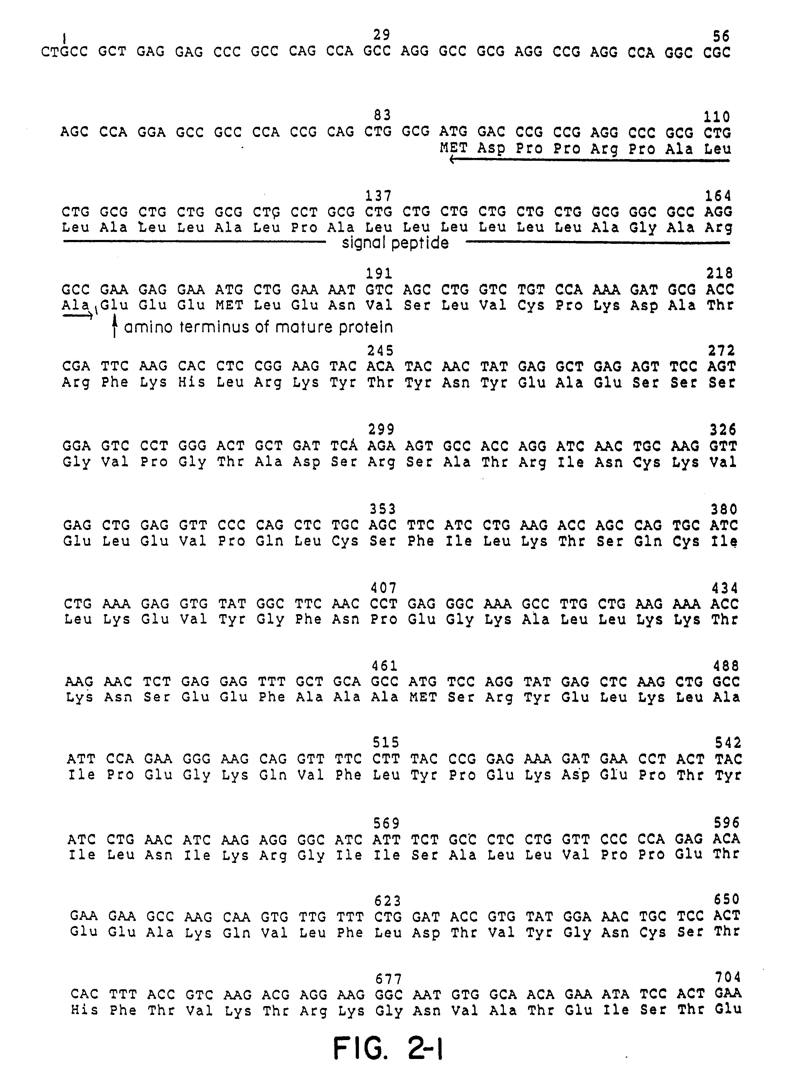

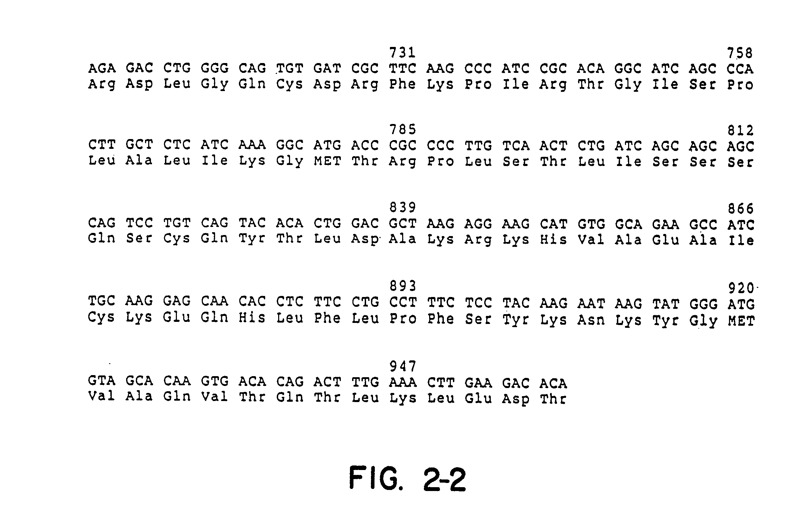

The 5'-apoB probe, disclosed in copending application PCT/US86/02048, is a 0.97 kb EcoRI/EcoRI insert fragment which contains 70 bp of 5' untranslated region and 900 bp of sequence encoding the 30 kd protein. This probe, however, does not overlap either of the published apoB clones described by Deeb, S.S., or Lusis, A.J. (supra). Isolation of the EcoRI fragment used as probe is described by protter, A.A., et al, Proc Natl Acad Sci (USA) (in press), and the complete DNA sequence of the 5'-apoB probe is shown in Figure 2.

In addition, a 3.1 kb genomic DNA fragment designated 3'-apoB probe is described by Scott, et al, Science (1985) 230: 37-43. It extends from an internal EcoRI site to almost the 3′ end of the gene.

The apoCl probe is a 300 bp cDNA with the sequence shown spanning nucleotide #50 to nucleotide #352 in the sequence of apoCl cDNA, by Knott, et al, Nucleic Acids Res (1984) 12 :3909-3915 (supra), incorporated herein by reference.

The pLDLR-3 probe is a portion of the full length (5.1 kb) human low density lipoprotein receptor-encoding DNA of Yamamoto et al, Cell (1984) 39: 27-38 (supra), incorporated herein by reference. The probe was prepared by cutting the full-length DNA fragment with XbaI and SmaI. This generates a 2.8 kb fragment from the 5′ end and eliminates repetitive sequences located in the 3′ untranslated region that would otherwise interfere with the hybridization (this probe was a gift from Yamamoto).

F.2 Atherosclerosis-Associated Polymorphisms

The XmnI/8.2 kb polymorphism has been shown to be highly predictive of atherosclerosis, and has been shown to be a 300 bp deletion about 4 kb 5′ of the apoAI gene. The polymorphism can be detected using XmnI digestion and probing with either apoAI probe or with the p5′AI probe; the presence of an 8.2 kb fragment shows the polymorphism is present. The same polymorphism can also be detected with other protocols, and the pattern which results depends on the restriction enzyme chosen and probe used to detect the digested fragments, as shown in the summary of Table 1. Table 1 shows the results obtained when DNA obtained from individuals heterozygous for the XmnI/8.2 kb polymorphism were digested with series of additional enzymes and probed with p5′AI probe. The table shows the length of the fragment associated with the normal genotype, and the fragment length obtained for the polymorphism.

## Table 1

### Additional Procedures to Detect
### the Atherosclerosis Deletion

| Probe | Restriction Enzyme | More Common Allele (kb) | Less Common Allele (kb) |
|-------|--------------------|-----------------------|------------------------|
| apoAI or p5′AI | XmnI | 8.5 | 8.2 |
| p5′AI | ApaI | 3.5 | 3.2 |
| p5′AI | BglI | 6.2 | 5.9 |
| p5′AI | BstEII | 2.5 | 2.1 |
| p5′AI | MspI | 4.0 | 5.9 |
| p5′AI | PvuII | 1.9 | 1.6 |
| p5′AI | RsaI | 3.5 | 3.2 |
| p5′AI | StuI | 5.8 | 5.5 |
| p5′AI | TaqI | 5.2 | 9.1 |
| p5′AI | BglII | 3.2 | 2.9 |
| p5′AI | HindIII | 2.7 | 2.4 |
| p5′AI | KpnI | 4.9 | 4.6 |
| p5′AI | MstII | 5.7 | 5.4 |
| p5′AI | NcoI | 4.5 | 4.2 |
| p5′AI | SspI | 2.8 | 2.5 |

The presence of the polymorphism which results in the generation of an 8.2 kb fragment upon XmnI digestion (or the results indicated on Table 1) correlates significantly with atherosclerosis. As described above, all individuals exhibiting this polymorphism in their genomes are not only affected by atherosclerosis but also exhibit severe clinical symptoms. However, the converse is not true: atherosclerosis may occur in individuals who lack this genetic abnormality. None of the individuals tested were homozygous with respect to this genetic feature; all contained an allele which generated the "normal" 8.5 kb fragment.

Additional polymorphisms can also be found which correlate in some way with the risk of atherosclerosis. To determine these polymorphisms, control and patient groups are set up using as a criterion positive or negative results relating to atheromatous plaque formation as determined by angiography. Persons are classified as "patients" who show plaques in this assay, whether or not they have had suffered heart attacks. They are designated "controls" if the results of this test are negative; none of these persons will have had a heart attack.

In interpreting the results, a standard x-square analysis is used to determine a significance level. The significance level represents the probability that the results obtained would not hold up if high numbers of subjects were used. For example, a significance level of less than 0.05 means that there is a greater than 95% probability that testing additional or larger numbers of subjects would yield the same results; a significance level of 0.10 means that there is one chance in 10 that the results would be different if a larger or different sample were tested.

The findings are interpreted in terms of the relative risk of persons having the polymorphism to show the disease, compared to those having an absence of the polymorphism. These "relative incidence" values are calculated according to Wolf, B., Ann Hum Genet (1955) 19:251. As applied to the assays below, the relative incidence is calculated as equal to:

$$\frac{PP \ x \ CN}{PN \ x \ CP}$$

where
PP is the number of patients having the polymorphism;
PN is the number of patients not having the polymorphism;
CP is the number of controls having the polymorphism;
CN is the number of controls not having the polymorphism.

The value calculated by this ratio, if significantly greater than 1, indicates that the persons having the polymorphism are at a greater risk of having the disease; a value less than 1 shows protection against the disease.

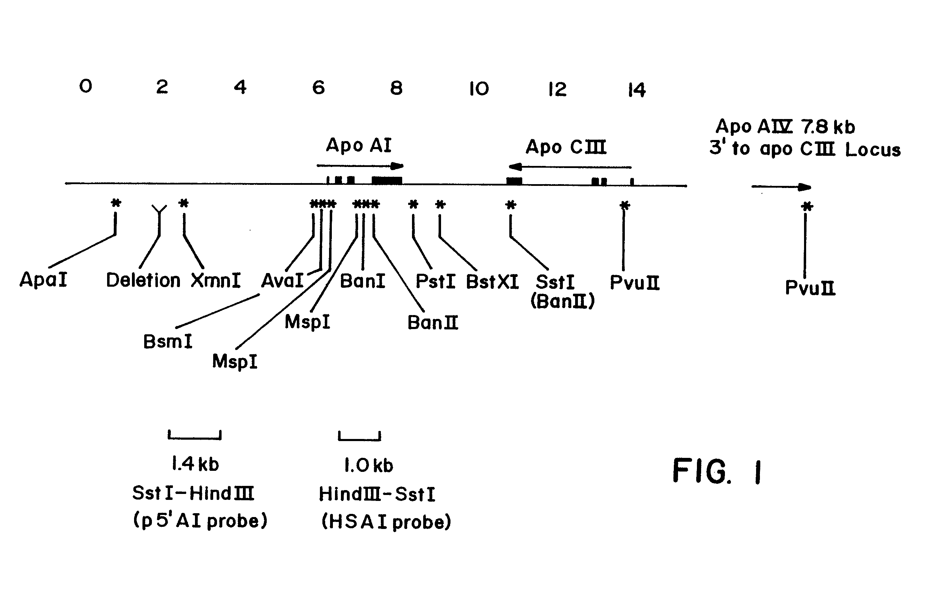

In addition to the deletion associated with atherosclerosis and heart attack outlined above, the polymorphisms of the invention are also present in the apoAI-CIII-AIV gene complex or apoAII, apoB, apoCI or LDL-R genes. The general pattern of the polymorphisms in the apoAI-CIII-AIV complex region is shown in Figure 1.

Table 2 shows numerous polymorphisms, including those of the invention, associated with apoAI/CIII/AIV; apoAII, apoB, apoCI, and LDL-R.

Table 2

| Polymorphism | Location of Polymorphism | Probe | Enzyme | More Common Allele, kb | Less Common Allele, kb | Freq.** |
|---|---|---|---|---|---|---|
| XmnI/8.2 kb | 4 kb 5' of apoAI gene | p5'AI apoAI[1] | XmnI | 8.5 | 8.2 | 0.03 ± 0.01 |
| BanI-apoAI | 3rd intron of apoAI gene | apoAI[1] | BanI | 0.82 0.64 | 1.46 | 0.16 ± 0.02 |
| Msp/0.73-apoAI | 1st intron of apoAI gene, 40 bp inside | apoAI[1] | MspI | 0.65 | 0.73 | 0.25 ± 0.02 |
| BanII-apoAI | 3rd intron of apoAI gene | apoAI[2] | BanII | 0.274 | 0.452 | 0.09 ± 0.02 |
| BsmI-apoAI | 3rd intron of apoAI gene | apoCIII apoAI[1] | BsmI | -- 1.3,5.6 | 9.3 6.9 | 0.05 ± 0.01 0.05 ± 0.01 |
| BstXI-apoAI | 1.0 kb 3' of apoAI gene | apoCIII, apoAI[1] | BstXI | 1.8 2.7 | 4.5 4.5 | 0.15 ± 0.02 0.15 ± 0.02 |
| BstEII-apoAII | -- | apoAII | BstEII | 6.5 | 4.8 | 0.05 ± 0.04 |
| HaeIII-apoB | -- | 3'-apoB | HaeIII | -- | 0.9-1.1 | -- |
| MvaI-apoB | 3' end of apoB gene | 3'-apoB | MvaI | 0.76 | 0.96 | 0.04 ± 0.01 |
| NsiI-apoB | -- | 5'-apoB | NsiI | 6.2 | 3.8 | 0.10 ± 0.01 |
| HpaI-apoB | -- | 5'-apoB | HpaI | -- | 15.1 | 0.05 ± 0.01 |
| BglI-apoCI | -- | apoCI | BglI | 6.2 | 8.7 | 0.03 ± 0.01 |
| DraI-apoCI | -- | apoCI | DraI | 7.6,2.6 | 10.2 | 0.22 ± 0.02 |
| TaqI-apoCI | -- | apoCI | TaqI | -- | 5.9 | 0.05 ± 0.01 |
| CfrBI-LDL-R | -- | pLDLR-3 | Cfr13I | 4.0 | 3.0 | 0.10 ± 0.02 |
| BstEII-LDL-R | -- | pLDLR-3 | BstEII | 33 | 22,11 | 0.25 ± 0.05 |

[1] first apoAI probe
[2] second apoAI probe, or "HSAI" probe
** error calculated as standard error of the mean

The polymorphisms of the invention tabulated above are as follows:

The BanI-apoAI polymorphism of the apoI gene is located in the third intron of the apoAI gene, about 260 bp downstream of the "MspI" polymorphism shown on the map of Figure 1. The majority (84%) of genomic DNA alleles digested with BanI and probed with apoAI probe, show a pair of fragments of 0.82 kb and 0.64 kb. A small proportion (16%) give a single 1.46 kb fragment.

The MspI/0.73-apoAI polymorphism of the apoAI gene is located in the first intron of the apoI gene (previously believed to be located in the 3'untranslated region; see U.S. Serial No. 924,911), about 40 bp inside of the beginning of the gene as shown on the map of Figure 1. The majority (75%) of genomic DNA alleles digested with MspI and probed with apoAI probe, show a fragment of 0.65 kb. A small proportion (25%) give a 0.73 kb fragment.

The BanII-apoAI polymorphism of the apoAI gene is located in the third intron of the apoAI gene, about 140 bp downstream from the BanI-apoAI polymorphism. The majority (91%) of genomic DNA alleles digested with BanII and probed with the HSAI (ApoAI[2]) probe described above show a fragment of 0.274 kb. A small proportion (9%) give a 0.452 kb fragment.

The BsmI-apoAI polymorphism of the apoAI gene is located in the third intron of the apoAI gene about 200 bp 3' of the Msp-apoAI polymorphism. It is detected against a constant background of a 18.0 kb fragment. The majority (95%) of genomic DNA alleles digested with BsmI and probed with apoAI[1] probe showed a fragment of 5.6 kb, while a small proportion (5%) gave a 6.9 kb fragment. When apoCIII is used as probe, 5% of the alleles yield a 9.3 kb fragment absent in the remaining 95%.

The BstXI-apoAI polymorphism of the apoAI gene is located about 1.0 kb 3' of the apoAI gene and detected against a constant background of 0.8 kb and 1.2 kb. The majority (85%) of genomic DNA alleles digested with BstXI and probed with apoCIII probe showed a 1.8 kb fragment, while a smaller proportion (15%) gave a 4.5 kb fragment. When ApoAI[1] is used as probe, the 95% of alleles give a 2.7 kb fragment, the remaining 15% a 4.5 kb fragment.

The BstEII-apoAII polymorphism was detected against a constant background of one 3.5 kb fragment, using a BstEII digest and the apoAII probe. A small proportion (4%) of the genomic DNA alleles digested with BstEII showed a 4.8 kb fragment, while the majority of alleles (96%) showed a 6.5 kb fragment.

The HaeIII-apoB polymorphism was detected against a constant background of 1.2 kb and 0.8 kb fragments, using a HaeIII digest. A small fraction (13%) of the genomic DNA alleles digested with HaeIII and probed with 3'-apoB probe showed a fragment of 0.9, while 7.1% showed a band at 1.0 kb and 16% showed a band at 1,1 kb.

The MvaI-apoB polymorphism was detected against a constant background of fragments of 0.56 kb, 0.87 kb and 1.10 kb using a MvaI digest and 3'-apoB probe. The majority (96%) of genomic DNA alleles showed a fragment of 0.76 kb. A smaller proportion (4%) provided a 0.96 kb fragment.

The NsiI-apoB polymorphism was detected against a constant background of an 8.9 kb fragment. The majority (90%) of genomic DNA alleles digested with NsiI and probed with apoB probe showed a 6.2 kb band, while a small proportion (10%) gave a 3.8 kb fragment.

The HpaI-apoB polymorphism was detected against a constant background of fragments of 2.1 kb, 4.5 kb, 8.1 kb and 13.0 kb. The majority (95%) of genomic DNA alleles digested with HpaI and probed with 5'-apoB probe showed no additional bands. A small proportion (5%) gave a 15.1 kb fragment.

The BglI-apoCI polymorphism of the apoCI gene was detected using a BglI digest. The majority (97.5%) of genomic DNA alleles digested with BglI and probed with apoCI probe, showed a fragment of 6.2 kb. A small proportion (2.5%) gave a 8.7 kb fragment.

The DraI-apoCI polymorphism of the apoCI gene was detected against a constant background of fragments of 2.4 kb, 3.5 kb, and 7.0 kb, using a DraI digest. The majority (74%) of genomic DNA alleles digested with DraI and probed with apoCI probe, showed a fragment of 7.6 kb and 2.6 kb. A smaller proportion (26%) gave a 10.2 kb fragment.

The TaqI-apoCI polymorphism of the apoCI gene was detected against a constant background of fragments of 0.9 kb. 1.7 kb, and 3.6 kb, using a TaqI digest. The majority (95%) of genomic DNA alleles digested with TaqI and probed with apoCI probe, showed no additional distinct bands. A small proportion (5%) gave a 5.9 kb fragment.

The Cfr13I-LDL receptor polymorphism was detected against a constant background of fragments using a Cfr13I digest. The majority (90%) of the genomic DNA alleles digested with Cfr13I and probed with the PLDLR-3 probe showed an additional 4.0 kb fragment. A small proportion (10%) gave an additional 3.0 kb fragment.

The BstEII-LDL receptor polymorphism was detected against a constant background of fragments using a BstEII digest. The majority (75%) of the genomic DNA alleles digested with BstEII and probed with the PLDLR-3 probe showed an additional 33 kb fragment. A smaller proportion (25%) showed two additional fragments of 22 kb and 11 kb.

The apoAI-CIII-AIV complex was screened with a number of enzymes which do not yield polymorphisms.

Specifically, when the 600 bp partial apoAI cDNA probe was used in the procedure of paragraph A, no polymorphism was found when the DNA had been digested with any of the following enzymes: ApaI, AvaI, AvaII, BamHI, BcII, BgII, BgIII, BstNI, EcoRI, EcoRV, HaeIII, HincII, HinfI, HphI, NciI, NcoI, PvuII, RsaI, ScaI, ScrFI, StuI, TaqI, and TthIIII.

When the p5'AI probe was used, no polymorphisms were obtained with AvaI, AvaII, BamHI, BanI, BanII, BcII, BstNI, EcoRI, EcoRV, HaeIII, HincIII, HinfI, HphI, NciI, pstI, ScaI, and SstI.

When apoCIII probe is used, no polymorphisms are found using AvaII, BgII, BstEII, HinfI, NcoI, pstI, RsaI, StuI, and TaqI.)

By testing a particular individual with respect to a series of polymorphisms, such as with the restriction enzymes and probes shown in Table 2, in addition to those otherwise known in the art, a genetic characterization (pattern) of this individual is obtained for genes associated with lipid transport. Determination of such genetic fingerprints is a tool for assigning familial relationships and heredity patterns. Such patterns are useful in determining parentage and tracing the incidence of gene-related disorders, and in predicting the occurrence of lipid transport/metabolism disorders.

Some of the listed polymorphisms in Table 2 have been located precisely; all are detectable using the procedures disclosed herein.

## Claims

1. A method for genetic analysis of an individual human subject which comprises detecting the presence or absence of at least one polymorphism selected from the group consisting of:

the BanI polymorphism of the apoAI gene,
The MspI/0.73 polymorphism of the apoAI gene,
the BanII polymorphism of the apoAI gene,
the BsmI polymorphism of the apoAI gene,
the BstXI polymorphism of the apoAI gene;

the BstEII polymorphism of the apoAII gene;

the HaeIII polymorphism of the apoB gene,
the MvaI polymorphism of the apoB gene,
the NsiI polymorphism of the apoB gene,
the HpaI polymorphism of the apoB gene;

the BglI polymorphism of the apoCI gene,
the DraI polymorphism of the apoCI gene,
the TaqI polymorphism of the apoCI gene;

the Cfr13I polymorphism of the LDL-R gene, and
the BstEII polymorphism of the LDL-R gene.

2. The method of claim 1 which comprises detecting the presence or absence of a diagnostic length DNA fragment of human genomic DNA digested with a restriction endonuclease, said fragment hybridizing to a detecting probe.

3. The method of claim 2 wherein at least one combination of detecting probe, restriction endonuclease, and diagnostic length DNA fragment is selected from the group consisting of:

apoAI[1] or substantially equivalent probe, BanI, and 1.46 kb;
apoAI[1] or substantially equivalent probe, MspI, and 0.73 kb;
apoAI[2] or substantially equivalent probe, BanII, and 0.45 kb;
apoAI[1] or substantially equivalent probe, BsmI, and 6.9 kb, or apoCIII or substantially equivalent probe. BsmI, and 9.3 kb;
apoCIII or substantially equivalent probe, BstXI, and 4.5 kb, or apoAI[1] or substantially equivalent probe, BstXI, and 4.5 kb;
apoAI[1] or substantially equivalent probe, BstEII and 4.8 kb;
3-apoB or substantially equivalent probe, HaeIII, and 0.9, 1.0 or 1.1 kb;
3-apoB or substantially equivalent probe, MvaI, and 0.96 kb;
5-apoB or substantially equivalent probe, NsiI, and 3.8 kb;
5-apoB or substantially equivalent probe, HpaI, and 15.1 kb;
apoCI or substantially equivalent probe, BglI and 8.7 kb;
apoCI or substantially equivalent probe, DraI, and 10.2 kb;
apoCI or substantially equivalent probe, TaqI, and 5.9 kb;
pLDLR-3 or substantially equivalent probe, Cfr13I, and 3.0 kb; and
pLDLR-3 or substantially equivalent probe, BstEII, and 22 and 11 kb.

4. A reagent kit useful in performing the method of claim 1, which kit includes at least one combination of probe and restriction enzyme selected from the group consisting of:

apoAI[1] or its substantial equivalent and BanI, MspI, BsmI, or BstXI;
apoAI[2] or its substantial equivalent and BanII;
apoCIII or its substantial equivalent and BsmI or BstXI;
apoAII or its substantial equivalent and BstEII;
3'-apoB or its substantial equivalent and HaeIII or MvaI;
5'-apoB or its substantial equivalent and NsiI or HpaI;
apoCI or its substantial equivalent and BglI, DraI or TaqI; and
pLDLR-3 or its substantial equivalent and Cfr13I or BstEII.

FIG. 1

```
                                    29                                      56
CTGCC GCT GAG GAG CCC GCC CAG CCA GCC AGG GCC GCG AGG CCG AGG CCA GGC CGC


                                    83                                     110
AGC CCA GGA GCC GCC CCA CCG CAG CTG GCG ATG GAC CCG CCG AGG CCC GCG CTG
                                             MET Asp Pro Pro Arg Pro Ala Leu
                                             ←

                                   137                                     164
CTG GCG CTG CTG GCG CTG CCT GCG CTG CTG CTG CTG CTG CTG GCG GGC GCC AGG
Leu Ala Leu Leu Ala Leu Pro Ala Leu Leu Leu Leu Leu Leu Ala Gly Ala Arg
————————————————————————————— signal peptide —————————————————————————

                                   191                                     218
GCC GAA GAG GAA ATG CTG GAA AAT GTC AGC CTG GTC TGT CCA AAA GAT GCG ACC
Ala Glu Glu Glu MET Leu Glu Asn Val Ser Leu Val Cys Pro Lys Asp Ala Thr
 →   ↑ amino terminus of mature protein

                                   245                                     272
CGA TTC AAG CAC CTC CGG AAG TAC ACA TAC AAC TAT GAG GCT GAG AGT TCC AGT
Arg Phe Lys His Leu Arg Lys Tyr Thr Tyr Asn Tyr Glu Ala Glu Ser Ser Ser


                                   299                                     326
GGA GTC CCT GGG ACT GCT GAT TCA AGA AGT GCC ACC AGG ATC AAC TGC AAG GTT
Gly Val Pro Gly Thr Ala Asp Ser Arg Ser Ala Thr Arg Ile Asn Cys Lys Val


                                   353                                     380
GAG CTG GAG GTT CCC CAG CTC TGC AGC TTC ATC CTG AAG ACC AGC CAG TGC ATC
Glu Leu Glu Val Pro Gln Leu Cys Ser Phe Ile Leu Lys Thr Ser Gln Cys Ile


                                   407                                     434
CTG AAA GAG GTG TAT GGC TTC AAC CCT GAG GGC AAA GCC TTG CTG AAG AAA ACC
Leu Lys Glu Val Tyr Gly Phe Asn Pro Glu Gly Lys Ala Leu Leu Lys Lys Thr


                                   461                                     488
AAG AAC TCT GAG GAG TTT GCT GCA GCC ATG TCC AGG TAT GAG CTC AAG CTG GCC
Lys Asn Ser Glu Glu Phe Ala Ala Ala MET Ser Arg Tyr Glu Leu Lys Leu Ala


                                   515                                     542
ATT CCA GAA GGG AAG CAG GTT TTC CTT TAC CCG GAG AAA GAT GAA CCT ACT TAC
Ile Pro Glu Gly Lys Gln Val Phe Leu Tyr Pro Glu Lys Asp Glu Pro Thr Tyr


                                   569                                     596
ATC CTG AAC ATC AAG AGG GGC ATC ATT TCT GCC CTC CTG GTT CCC CCA GAG ACA
Ile Leu Asn Ile Lys Arg Gly Ile Ile Ser Ala Leu Leu Val Pro Pro Glu Thr


                                   623                                     650
GAA GAA GCC AAG CAA GTG TTG TTT CTG GAT ACC GTG TAT GGA AAC TGC TCC ACT
Glu Glu Ala Lys Gln Val Leu Phe Leu Asp Thr Val Tyr Gly Asn Cys Ser Thr


                                   677                                     704
CAC TTT ACC GTC AAG ACG AGG AAG GGC AAT GTG GCA ACA GAA ATA TCC ACT GAA
His Phe Thr Val Lys Thr Arg Lys Gly Asn Val Ala Thr Glu Ile Ser Thr Glu
```

## FIG. 2-1

```
                                                731                                        758
AGA GAC CTG GGG CAG TGT GAT CGC TTC AAG CCC ATC CGC ACA GGC ATC AGC CCA
Arg Asp Leu Gly Gln Cys Asp Arg Phe Lys Pro Ile Arg Thr Gly Ile Ser Pro


                                                785                                        812
CTT GCT CTC ATC AAA GGC ATG ACC CGC CCC TTG TCA ACT CTG ATC AGC AGC AGC
Leu Ala Leu Ile Lys Gly MET Thr Arg Pro Leu Ser Thr Leu Ile Ser Ser Ser


                                                839                                        866
CAG TCC TGT CAG TAC ACA CTG GAC GCT AAG AGG AAG CAT GTG GCA GAA GCC ATC
Gln Ser Cys Gln Tyr Thr Leu Asp Ala Lys Arg Lys His Val Ala Glu Ala Ile


                                                893                                        920
TGC AAG GAG CAA CAC CTC TTC CTG CCT TTC TCC TAC AAG AAT AAG TAT GGG ATG
Cys Lys Glu Gln His Leu Phe Leu Pro Phe Ser Tyr Lys Asn Lys Tyr Gly MET


                                                947
GTA GCA CAA GTG ACA CAG ACT TTG AAA CTT GAA GAC ACA
Val Ala Gln Val Thr Gln Thr Leu Lys Leu Glu Asp Thr
```

# FIG. 2-2